(19) Europäisches Patentamt — European Patent Office — Office européen des brevets

(11) **EP 4 115 180 B1**

(12) **EUROPÄISCHE PATENTSCHRIFT**

(45) Veröffentlichungstag und Bekanntmachung des Hinweises auf die Patenterteilung:
**06.05.2026  Patentblatt 2026/19**

(21) Anmeldenummer: **21709922.5**

(22) Anmeldetag: **02.03.2021**

(51) Internationale Patentklassifikation (IPC):
**G01N 33/497** *(2006.01)*     **G01N 33/00** *(2006.01)*

(52) Gemeinsame Patentklassifikation (CPC):
**A61B 5/082; G01N 33/4975;** G01N 33/0029;
Y02A 50/20

(86) Internationale Anmeldenummer:
**PCT/EP2021/055115**

(87) Internationale Veröffentlichungsnummer:
**WO 2021/175812 (10.09.2021 Gazette 2021/36)**

(54) **REINIGUNGSVERFAHREN EINES SENSORS IN EINEM ATEMGASANALYSEGERÄTS**

CLEANING METHOD FOR A SENSOR IN A RESPIRATORY GAS ANALYSIS DEVICE

PROCÉDÉ DE NETTOYAGE D'UN CAPTEUR DANS UN DISPOSITIF D'ANALYSE DE GAZ RESPIRATOIRE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **05.03.2020  DE 102020202798**

(43) Veröffentlichungstag der Anmeldung:
**11.01.2023  Patentblatt 2023/02**

(73) Patentinhaber: **Robert Bosch GmbH**
**70442 Stuttgart (DE)**

(72) Erfinder:
• **BECK, Christoph**
  **73732 Esslingen (DE)**
• **JANK, Heike**
  **71394 Kernen Im Remstal (DE)**
• **THUERSAM, Markus**
  **71263 Weil Der Stadt (DE)**
• **SCHECK, Kathrin**
  **71334 Waiblingen (DE)**

(56) Entgegenhaltungen:
**EP-A2- 0 488 102     DE-A1- 102011 003 291
DE-A1- 4 107 221**

**Beschreibung**

Die vorliegende Erfindung betrifft ein Verfahren zum Betreiben eines Atemgasanalysegeräts. Weiterhin betrifft die vorliegende Erfindung ein Atemgasanalysegerät das zur Durchführung des Verfahrens eingerichtet ist.

Stand der Technik

Die von einem Menschen ausgeatmete Luft (Ausatemluft) enthält verschiedene medizinisch und insbesondere diagnostisch interessante Substanzen (Biomarker). So kann beispielsweise der Stickstoffmonoxidgehalt (fractional exhaled nitric oxide, FeNO) in der Ausatemluft untersucht werden, um Entzündungsprozesse oder chronische pulmonale Erkrankungen, wie beispielsweise Asthma bronchiale, erkennen zu können. Zur Analyse der Ausatemluft sind Atemgasanalysegeräte bekannt, die über entsprechende Sensoren, insbesondere Gassensoren, verfügen. Hierbei kommen vor allem Stickstoffmonoxid-Sensoren und/oder Stickstoffdioxid-Sensoren zum Einsatz, die für die Konzentrationsmessungen dieser Substanzen in der Ausatemluft genutzt werden. Derartige Gassensoren sind in der Regel empfindlich gegenüber Quereinflüssen, sodass sich beispielsweise variable Umgebungsfeuchtigkeiten und Umgebungstemperaturen auf die Messgenauigkeit auswirken können. Somit kann es auch zu Lagereffekten an den Gassensoren kommen, wobei sich die Empfindlichkeit des Sensors mit andauernder Lagerung in Abhängigkeit von den Lagerbedingungen ändert und auf das Messsignal auswirkt. In diesem Zusammenhang spielt insbesondere die Feuchtigkeit, die sich am Sensor einstellt, eine Rolle. Des Weiteren wird der Zustand und damit die Baseline eines Gassensors auch durch die eigentliche Gasmessung verändert, da beispielsweise die sensitive Schicht des Gassensors durch die Beaufschlagung des Gases während der Messphase belastet bzw. verschmutzt wird.

Die DE 10 2011 003 291 A1 beschreibt ein Betriebsverfahren für eine Gassensor zur Ermittlung der Stickstoffmonoxid- oder Stickstoffdioxidkonzentration, bei dem zwischen einer Messphase und einer Regenerationsphase gewechselt wird, wobei während der Messphase das zu vermessende Gas dem Gassensor zugeleitet wird, und während der Regenerationsphase ein Gas mit geringerer Konzentration an Stickstoffmonoxid oder Stickstoffdioxid zum Gassensor geleitet wird. Während der Regenerationsphase wird der Gassensor mittels einer Heizvorrichtung auf eine Temperatur oberhalb der Messtemperatur aufgeheizt.

In der DE 41 07 221 A1 wird ein Verfahren zur Bestimmung einer Gaskonzentration mit Hilfe von gasempfindlichen Halbleitern beschrieben. Nach Erreichen eines vorgegebenen Schwellwertes für eine Leitwertzunahme wird ein Messzyklus gestartet wird, bei dem bis zum Erreichen eines weiteren Schwellwertes die Zeit gemessen wird, was zur Folge hat, dass dann eine Temperaturerhöhung des Substrates bewirkt wird. Die Ausheizzeit ist eine Funktion der Zeit. Dabei kann die Ausheizzeit in etwa umgekehrt proportional zur Messzeit sein. Alternativ wird die Ausheizzeit um einen stetig sich vergrößern den Faktor verlängert, wenn in einer Folge von Messzyklen der Schwellwert nicht wieder erreicht wird.

Aus der EP 0 488 102 A2 ist ein Verfahren zur Gasmessung bekannt, in dem ein Halbleitersensorelement eingesetzt wird. Die Temperatur des Halbleitersensorelements wird gemessen. Sein Leitwert bzw. Widerstand ist durch das Messgas beeinflussbar. Um bei dem Verfahren bessere und genauere Messergebnisse zu erzielen, werden die Temperatur und/oder der Leitwert bzw. Widerstand und/oder die Bedeckung mit Messgas des Halbleitersensorelements durch Eingriffe beeinflusst und/oder als Messgröße erfasst.

Offenbarung der Erfindung

Das Verfahren zum Betreiben eines Atemgasanalysegeräts mit wenigstens einem Gassensor sieht vor, dass nach Durchführung einer Atemgasanalyse der Gassensor auf eine Temperatur erhitzt wird, die über einem vorgebbaren Temperaturschwellenwert liegt. Dieser Temperaturschwellenwert beträgt vorzugsweise wenigstens 100°C. Weiter ist bevorzugt, dass der Gassensor beim Erhitzen mit einem Spülgas überströmt wird.

Dieses Verfahren hat den Vorteil, dass es eine Regeneration des Gassensors durch Desorption der während der Messung adsorbierten Gasmoleküle sowie ein Austreiben von daran adsorbierter Feuchtigkeit ermöglicht und so seine Messgenauigkeit über die Lebenszeit des Atemgasanalysegeräts sicherstellt. Ein Austausch oder eine Nachkalibrierung des Gassensors ist dann nicht notwendig. Falls das Atemgasanalysegerät vor Durchführung der Atemgasanalyse eine Prä-Regeneration vorsieht, in der durch ein Aufheizen des Gassensors unmittelbar vor Durchführung der Atemgasanalyse Störeinflüsse aus der Umgebung eliminiert werden und ein Aufwärmen auf eine Betriebstemperatur erfolgt, sodass vor Start einer Messung ein definierter Sensorzustand erreicht werden kann, so ermöglicht es das Verfahren, die Prä-Regeneration zu verkürzen oder sogar ganz auf diese zu verzichten.

Bei dem Gassensor kann es sich insbesondere um einen Stickstoffmonoxidsensor oder um einen Stickstoffdioxidsensor handeln.

Unter der Durchführung der Atemgasanalyse wird der Zeitraum verstanden, in dem der Gassensor einem Atemgas eines Benutzers des Atemgasanalysegeräts ausgesetzt wird und sein Sensorsignal zur Analyse der Gaskomponente, auf welche der Gassensor empfindlich reagiert, ausgewertet werden kann. Unter bestimmten Umständen wird anstelle einer Atemgasanalyse eine Analyse der Umgebungsluft durchgeführt, wobei sich die zu bestimmende Gaskomponente nicht von der einer Atemgasanalyse unterscheitet.

Das Erhitzen wird durchgeführt, bis ein Abbruchkriterium erfüllt ist oder es ist eine Gruppe von mehreren Abbruchkriterien vorgesehen. Das Erhitzen wird dann so lange durchgeführt, bis alle Abbruchkriterien der Gruppe erfüllt sind. Das Erhitzen wird in Abhängigkeit vom Regenerationsverlauf am Gassensor beendet. Dies hat den Vorteil, dass die Heizphase gerätespezifisch und bedarfsgerecht für jede Messung festgelegt werden kann. Dies hat den Nebeneffekt, dass die Zeit bis zur erneuten Messbereitschaft des Atemgasanalysegeräts nicht pauschal festgelegt werden muss, sondern in Abhängigkeit des Zustands des Gassensors bedarfsgerecht gewählt und damit die benötigte Heizphase verkürzt werden kann. Dazu können verschiedene Abbruchkriterien herangezogen werden:

Ein Abbruchkriterium kann darin bestehen, dass eine Differenz oder der Betrag einer Differenz zwischen einem aktuellen Signal des Gassensors und einem Signal des Gassensors bei Beginn der Atemgasanalyse einen vorgegeben Differenzschwellenwert unterschreitet. Ist die Differenz äußerst gering, so ist dies ein Hinweis darauf, dass sich der Gassensor hinreichend regeneriert hat und für eine unmittelbare Folgemessung bereitsteht. Der Differenzschwellenwert wird vorzugsweise so gewählt, dass er im Bereich des Rauschens des Signals des Gassensors liegt.

Ein anderes geeignetes Abbruchkriterium kann überprüft werden, indem ein Quotient aus einer Differenz zwischen einem aktuellen Signal des Gassensors und einem Signal des Gassensors bei Beginn der Atemgasanalyse und einer Differenz zwischen einem Signal des Gassensors am Ende der Atemgasanalyse und dem Signal des Gassensors bei Beginn der Atemgasanalyse gebildet wird und mit einem vorgebbaren Quotientenschwellenwert verglichen wird. Das Abbruchkriterium ist erfüllt, wenn der Quotient den Quotientenschwellenwert unterschreitet. Am Ende der Atemgasanalyse beträgt der Quotient zunächst 1,00 und fällt dann im Verlauf der Regeneration. Wenn die Differenz zwischen dem aktuellen Signal des Gassensors und dem Signal des Gassensors zum Beginn der Atemgasanalyse relativ zur Differenz zwischen den Signalen des Gassensors am Ende und am Beginn der Atemgasanalyse sehr klein wird, weist dies auf eine ausreichende Regeneration hin. Hierzu weist der Quotientenschwellenwert vorzugsweise einen Wert von weniger als 0,05 auf.

Noch ein anderes geeignetes Abbruchkriterium besteht darin, dass ein Betrag eines Gradienten eines Signalverlaufs des Gassensors einen vorgebbaren Gradientenschwellenwert unterschreitet. Als Signalverlauf wird insbesondere der Signalverlauf mit der Zeit verwendet. Der Gradient kann dann als erste Ableitung des Signals nach der Zeit ausgedrückt werden. Er gibt eine Auskunft über die Veränderung des Sensorsignals. Ist diese Veränderung vernachlässigbar gering, so kann der Gassensor als hinreichend regeneriert betrachtet werden. Hierzu ist der Gradientenschwellenwert vorzugsweise kleiner als 1 % pro Sekunde.

Noch ein anderes geeignetes Abbruchkriterium besteht darin, dass ein Betrag einer mehrfachen Ableitung, insbesondere einer zweiten Ableitung, eines Signalverlaufs des Gassensors einen vorgebbaren Ableitungsschwellenwert unterschreitet. Hierdurch fließt die zeitliche Änderung der Signalsteigung oder der Signalkrümmung in die Auswertung mit ein. Je geringer diese Werte ausfallen, desto stabiler verhält sich das Signal, was auf eine hinreichende Regeneration des Gassensors hinweist.

Jedes der Abbruchkriterien wird im Zusammenhang mit einer Änderung der Temperatur des Gassensors oder einer Änderung eines über den Gassensor geleiteten Spülgasstroms oder einer Änderung der an den Gassensor angelegten elektrischen Spannung ausgewertet. Bei der Änderung des Spülgasstroms kann es sich insbesondere um eine Änderung des Volumenstroms und/oder um eine Änderung der Feuchte des Spülgasstroms und/oder um eine Änderung seiner Temperatur handeln. Diese Änderungen können insbesondere Sprünge des jeweiligen Parameters, rampenförmige Änderungen des Parameters oder periodische Modulationen, wie beispielsweise sinusförmige Modulationen oder rechteckförmige Modulationen sein. Bei einer Änderung der an den Gassensor angelegten elektrischen Spannung handelt es sich vorzugsweise ebenso um Sprünge, rampenförmige Änderungen oder periodische Modulationen der Spannung. Reagiert das Signal darauf mit einer signifikanten Änderung des betrachteten Merkmals, so ist dies ein Hinweis darauf, dass der Gassensor noch nicht ausreichend regeneriert ist. Reagiert das Merkmal hingegen gar nicht oder nur marginal auf die Änderung des Parameters, so befindet sich der Sensor in einem hinreichend regenerierten Zustand.

Die Prüfung auf Erfüllung des Abbruchkriteriums kann bevorzugt kontinuierlich oder in zeitlichen Abständen erfolgen. Die zeitlichen Abstände können regelmäßige Abstände sein. Alternativ erfolgt die Prüfung auf Erfüllung des Abbruchkriteriums oder der Gruppe von Abbruchkriterien allerdings in zeitlichen Abständen, die jeweils in Abhängigkeit von einem zuletzt ermittelten Wert mindestens eines Abbruchkriteriums gewählt werden. Hierdurch kann je nachdem wie weit ein untersuchtes Merkmal noch von seinem Schwellenwert entfernt ist, der nächste zeitliche Abstand geeignet wählt werden, sodass die Prüfung zunächst in langen zeitlichen Abständen erfolgt, die zum Ende der Regeneration hin kürzer werden. Ein Zusammenhang zwischen dem Abstand des untersuchten Merkmals von seinem Schwellenwert und dem nächsten zeitlichen Abstand kann insbesondere empirisch ermittelt werden.

Wenn bereits bei Beenden der Atemgasanalyse das Abbruchkriterium oder die Gruppe von Abbruchkriterien erfüllt ist, so ist es bevorzugt, dass das Erhitzen unterdrückt wird, da in diesem Fall eine Regeneration des Gassensors nicht erforderlich ist.

Das Atemgasanalysegerät ist zur Durchführung des Verfahrens eingerichtet und weist damit die auch für das Verfahren diskutierten Vorteile auf.

Kurze Beschreibung der Zeichnungen

Ausführungsbeispiele der Erfindung sind in den Zeichnungen dargestellt und werden in der nachfolgenden Beschreibung näher erläutert.

Figur 1 zeigt ein Ablaufdiagramm eines Ausführungsbeispiels des erfindungsgemäßen Verfahrens.
Figur 2 zeigt einen zeitlichen Signalverlauf eines Gassensors in einem Ausführungsbeispiel des erfindungsgemäßen Verfahrens.

Ausführungsbeispiele der Erfindung

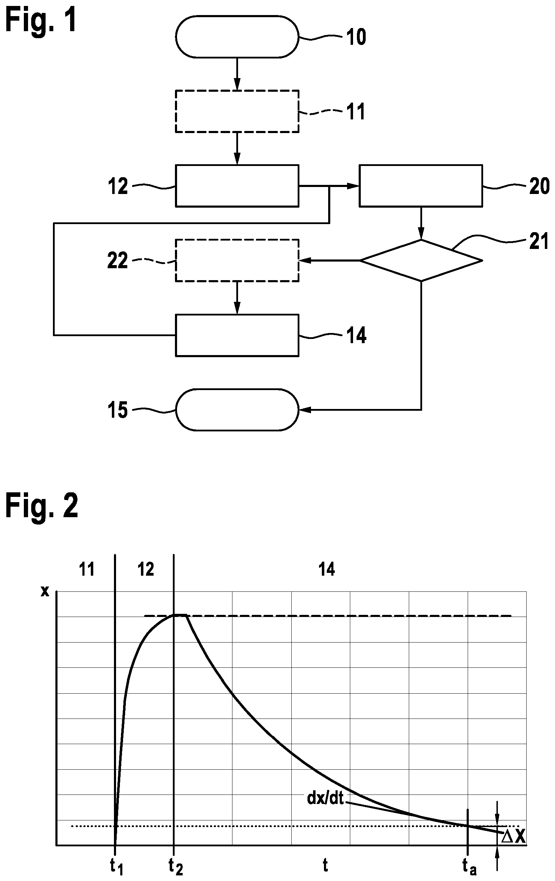

Ein Atemgasanalysegerät weist einen Gassensor auf, der in dem folgenden Ausführungsbeispiel der Erfindung beispielsweise als Stickstoffdioxidsensor ausgeführt sein kann. Soll damit der Stickstoffmonoxidgehalt der Ausatemluft bestimmt werden, wird hierzu ein Konverter vorgeschaltet, der beispielsweise in ein Mundstück integriert ist. In einem Ausführungsbeispiel der Erfindung wird das Atemgasanalysegerät dabei mittels eines Verfahrens betrieben, das in Figur 1 dargestellt ist. Nach dem Start 10 des Verfahrens wird in Abhängigkeit vom eingestellten Betriebsmodus des Atemgasanalysegeräts gegebenenfalls eine Prä-Regeneration 11 durchgeführt. Hierzu wird der Gassensor beheizt und mittels eines Spülgases gespült. Anschließend wird eine Atemgasanalyse 12 durchgeführt, indem Atemgas in das Atemgasanalysegerät eingeleitet wird und der Gassensor dabei mit der zu bestimmenden Gaskonzentration an Stickstoffdioxidbeaufschlagt wird. Die Gasmoleküle lagern sich an der sensitiven Schicht des Gassensors an und verändern somit dessen Potential. Anhand der Potentialänderung kann die gesuchte Konzentration an Stickstoffmonoxid oder Stickstoffdioxid in der Atemgasprobe bestimmt werden. Nach Beendigung der Atemgasanalyse 12 und somit nach Wegnahme der Gasbeaufschlagung verbleiben Gasmoleküle an der sensitiven Schicht und verunreinigen damit den Gassensor. In Folge dessen besitzt er nach einer durchgeführten Atemgasanalyse einen anderen Zustand, als noch vor der Messung. Die Sensitivität des Gassensors sinkt, womit auch seine Messgenauigkeit und die noch mögliche Anzahl an Messungen verringert werden. Ein anschließendes Erhitzen 14 stellt eine Post-Regeneration des Gassensors dar. Hierbei wird der Gassensor auf eine Temperatur von 100°C erhitzt und mit einem Spülgas überströmt. Nachdem der vorgegebene Zeitraum abgelaufen ist, werden die Post-Regenerationsmaßnahmen beendet und es erfolgt ein Beenden 15 des Verfahrens.

Figur 2 zeigt den Verlauf des Signals x mit der Zeit t, während der Prä-Regeneration 11, der Atemgasanalyse 12 und dem Erhitzen 14 der Post-Regeneration. Unter Erhitzen wird im Folgenden sowohl das Aufheizen auf eine vorgegebene Temperatur als auch das Halten auf dieser Temperatur verstanden, um so ein Ausheizen des Gassensors zu erreichen. Dabei wird das Signal x als elektrisches Potential gemessen. Bei der Prä-Regeneration 11 wird ein stabiles Ausgangsniveau des Signals x erreicht. Zum Zeitpunkt $t_1$ steigt das Signal x mit Beginn der Atemgasanalyse 12 sprunghaft an. Am Ende der Atemgasanalyse 12 zum Zeitpunkt $t_2$ erreicht es sein Maximum. Anschließend sinkt es dann während des Erhitzens 14 des Gassensors wieder ab.

An die Atemgasanalyse 12 schließt sich die Ermittlung 20 von einem oder mehreren Merkmalen von Abbruchkriterien an. In einer anschließenden Prüfung 21 wird geprüft, ob alle, den Merkmalen zugeordneten Abbruchkriterien erfüllt sind. Ist dies der Fall, so wird das Erhitzen 14 unterdrückt und es erfolgt ein sofortiges Beenden 15 des Verfahrens. Andernfalls wird für das Erhitzen ein Zeitraum bis zur nächsten Prüfung auf die Erfüllung der Abbruchkriterien gewählt 22. Die Wahl 22 erfolgt in Abhängigkeit von den Werten der in Schritt 20 ermittelten Merkmale. Ein gewählter Zeitraum kann beispielsweise 30 Sekunden lang sein. Nachdem der vorgegebene Zeitraum abgelaufen ist, erfolgt ein erneutes Ermitteln 20 der Merkmale und eine erneute Prüfung 21. Dies wird solange fortgesetzt, bis alle Abbruchkriterien erfüllt sind und ein Beenden 15 des Verfahrens ohne weiteres Erhitzen 14 erfolgt. Dabei ergibt sich derselbe Verlauf des Signals x, wie er in Figur 2 dargestellt ist.

Ein erstes Abbruchkriterium besteht darin, dass eine Differenz $\Delta x$ einen Differenzschwellenwert unterschreitet. Diese wird gemäß Formel 1 ermittelt:

$$\Delta x = x(t_a) - x(t_1) \hspace{6cm} \text{(Formel 1)}$$

Dabei bezeichnet $x(t_a)$ das Signal x zum aktuellen Zeitpunkt $t_a$ der Durchführung des Schritts 20 und $x(t_1)$ bezeichnet das Signal x zum Zeitpunkt $t_1$ des Beginns der Atemgasanalyse 12.

Ein zweites Abbruchkriterium besteht darin, dass ein Quotient qx einen Quotientenschwellenwert unterschreitet.

Der Quotient qx wird gemäß Formel 2 berechnet:

$$qx = \frac{x(t_a) - x(t_1)}{x(t_2) - x(t_1)} = \frac{\Delta x}{x(t_2) - x(t_1)} \qquad \text{(Formel 2)}$$

Dabei sind $\Delta x$, $x(t_a)$ und $x(t_1)$ genauso wie in Formel 1 definiert. $x(t_2)$ bezeichnet das Signal zum Zeitpunkt $t_2$ am Ende der Atemgasanalyse 12.

Ein drittes Abbruchkriterium besteht darin, dass ein Betrag eines Gradienten dx/dt des Verlaufs des Signals x mit der Zeit t einen Gradientenschwellenwert unterschreitet. Wie in Figur 2 dargestellt ist, nimmt der Betrag des negativen Gradienten dx/dt zum Ende des Erhitzens 14 hin ab.

Ein viertes Abbruchkriterium besteht darin, dass ein Betrag einer zweiten Ableitung $d^2x/dt^2$ des Signals x nach der Zeit t einen Ableitungsschwellenwert unterschreitet.

Alle voranstehend genannten Merkmale, die für die Prüfung auf die Abbruchkriterien herangezogen werden, werden im vorliegenden Ausführungsbeispiel tiefpassgefiltert, um so hochfrequente Störungen und Signaltauschen herauszufiltern.

Beim Erhitzen 14 wird vor einer erneuten Ermittlung der Merkmale 20 eine sprunghafte Temperaturerhöhung des Gassensors durchgeführt. Hierzu wird die Temperatur vorliegend von 100°C auf 130°C erhöht. Reagiert mindestens eines der im Schritt 20 ermittelten Merkmale mit einer signifikanten Änderung von zumindest mehreren Prozent, so wird dies als Hinweis darauf ausgelegt, dass der Sensor noch nicht hinreichend regeneriert ist und unabhängig von der Erfüllung der Abbruchkriterien nach der Prüfung 21 noch kein Beenden 15 des Verfahrens erfolgen darf.

In Varianten des Ausführungsbeispiels erfolgt die Abfolge von Ermittlung 20 der Merkmale, Prüfung 22 und Erhitzen 14 kontinuierlich in so schneller Abfolge wie möglich, sodass die Wahl 22 eines definierten Zeitraums überflüssig wird.

Während des Erhitzens 14 des Gassensors wird das Atemgasanalysegerät in allen Ausführungsbeispielen des Verfahrens für weitere Messungen gesperrt.

## Patentansprüche

1. Verfahren zum Betreiben eines Atemgasanalysegeräts mit wenigstens einem Gassensor, wobei nach Durchführung einer Atemgasanalyse (12) der Gassensor auf eine Temperatur erhitzt wird (14), die über einem vorgebbaren Temperaturschwellenwert liegt, bis ein Abbruchkriterium oder eine Gruppe von mehreren Abbruchkriterien erfüllt ist, **dadurch gekennzeichnet, dass** jedes der Abbruchkriterien im Zusammenhang mit einer Änderung der Temperatur des Gassensors oder einer Änderung eines über den Gassensor geleiteten Spülgasstroms oder einer Änderung der an den Gassensor angelegten elektrischen Spannung ausgewertet wird.

2. Verfahren nach Anspruch 1, **dadurch gekennzeichnet, dass** ein Abbruchkriterium darin besteht, dass eine Differenz ($\Delta x$) zwischen einem aktuellen Signal ($x(t_a)$) des Gassensors und einem Signal ($x(t_1)$) des Gassensors bei Beginn der Atemgasanalyse oder ein Betrag dieser Differenz ($\Delta x$) einen vorgebbaren Differenzschwellenwert unterschreitet.

3. Verfahren nach Anspruch 1 oder 2, **dadurch gekennzeichnet, dass** ein Abbruchkriterium darin besteht, dass ein Quotient aus einer Differenz ($\Delta x$) zwischen einem aktuellen Signal ($x(t_a)$) des Gassensors und einem Signal ($x(t_1)$) des Gassensors bei Beginn der Atemgasanalyse und einer Differenz zwischen einem Signal ($x(t_2)$) des Gassensors am Ende der Atemgasanalyse und dem Signal ($x(t_1)$) des Gassensors bei Beginn der Atemgasanalyse einen vorgebbaren Quotientenschwellenwert unterschreitet.

4. Verfahren nach einem der Ansprüche 1 bis 3, **dadurch gekennzeichnet, dass** ein Abbruchkriterium darin besteht, dass ein Betrag eines Gradienten (dx/dt) eines Signalverlaufs des Gassensors einen vorgebbaren Gradientenschwellenwert unterschreitet.

5. Verfahren nach einem der Ansprüche 1 bis 4, **dadurch gekennzeichnet, dass** ein Abbruchkriterium darin besteht, dass ein Betrag einer mehrfachen Ableitung eines Signalverlaufs des Gassensors einen vorgebbaren Ableitungsschwellenwert unterschreitet.

6. Verfahren nach einem der Ansprüche 1 bis 5, **dadurch gekennzeichnet, dass** eine Prüfung (21) auf Erfüllung des Abbruchkriteriums oder der Gruppe von Abbruchkriterien in zeitlichen Abständen erfolgt, die jeweils in Abhängigkeit von einem zuletzt ermittelten Wert mindestens eines Abbruchkriteriums gewählt werden (22).

7. Verfahren nach einem der Ansprüche 1 bis 6, **dadurch gekennzeichnet, dass** das Erhitzen (14) unterdrückt wird, wenn bereits bei Beenden der Atemgasanalyse (12) das Abbruchkriterium oder der Gruppe von Abbruchkriterien erfüllt ist.

8. Atemgasanalysegerät das zur Durchführung eines Verfahrens gemäß einem der Ansprüche 1 bis 7 eingerichtet ist.

## Claims

1. Method for operating a respiratory gas analysis device having at least one gas sensor, wherein, after a respiratory gas analysis (12) has been carried out, the gas sensor is heated (14) to a temperature which is above a specifiable temperature threshold value until a termination criterion or a group of a plurality of termination criteria has been met, **characterized in that** that each of the termination criteria is evaluated in connection with a change in the temperature of the gas sensor or a change in a purge gas flow passed over the gas sensor or a change in the electrical voltage applied to the gas sensor.

2. Method according to Claim 1, **characterized in that** one termination criterion is that a difference ($\Delta x$) between a present signal ($x(t_a)$) of the gas sensor and a signal ($x(t_1)$) of the gas sensor at the beginning of the respiratory gas analysis or an absolute value of this difference ($\Delta x$) falls below a specifiable difference threshold value.

3. Method according to Claim 1 or 2, **characterized in that** one termination criterion is that a quotient of a difference ($\Delta x$) between a present signal ($x(t_a)$) of the gas sensor and a signal ($x(t_1)$) of the gas sensor at the beginning of the respiratory gas analysis and a difference between a signal ($x(t_2)$) of the gas sensor at the end of the respiratory gas analysis and the signal ($x(t_1)$) of the gas sensor at the beginning of the respiratory gas analysis falls below a specifiable quotient threshold value.

4. Method according to any of Claims 1 to 3, **characterized in that** one termination criterion is that an absolute value of a gradient (dx/dt) of a signal profile of the gas sensor falls below a specifiable gradient threshold value.

5. Method according to any of Claims 1 to 4, **characterized in that** one termination criterion is that an absolute value of a repeated derivative of a signal profile of the gas sensor falls below a specifiable derivative threshold value.

6. Method according to any of Claims 1 to 5, **characterized in that** a check (21) as to whether the termination criterion or the group of termination criteria has been met is performed at time intervals that are each chosen (22) depending on a last determined value of at least one termination criterion.

7. Method according to any of Claims 1 to 6, **characterized in that** the heating (14) is suppressed if the termination criterion or the group of termination criteria has already been met at the end of the respiratory gas analysis (12).

8. Respiratory gas analysis device configured for carrying out a method according to any of Claims 1 to 7.

## Revendications

1. Procédé pour faire fonctionner un appareil d'analyse de gaz respiratoire avec au moins un capteur de gaz, le capteur de gaz étant chauffé (14), après la réalisation d'une analyse de gaz respiratoire (12), à une température qui se situe au-dessus d'une valeur de seuil de température pouvant être spécifiée jusqu'à ce qu'un critère d'interruption ou un groupe de plusieurs critères d'interruption soit satisfait, **caractérisé en ce que** chacun des critères d'interruption est évalué en lien avec une variation de la température du capteur de gaz ou une variation d'un flux de gaz de rinçage acheminé par le capteur de gaz ou une variation de la tension électrique appliquée sur le capteur de gaz.

2. Procédé selon la revendication 1, **caractérisé en ce qu'**un critère d'interruption consiste **en ce qu'**une différence ($\Delta x$) entre un signal actuel ($x(t_a)$) du capteur de gaz et un signal ($x(t_1)$) du capteur de gaz au début de l'analyse de gaz respiratoire ou une valeur de ladite différence ($\Delta x$) ne dépasse pas une valeur de seuil de différence pouvant être spécifiée.

3. Procédé selon la revendication 1 ou 2, **caractérisé en ce qu'**un critère d'interruption consiste **en ce qu'**un quotient d'une différence ($\Delta x$) entre un signal actuel ($x(t_a)$) du capteur de gaz et un signal ($x(t_1)$) du capteur de gaz au début de

**EP 4 115 180 B1**

l'analyse de gaz respiratoire et d'une différence entre un signal ($x(t_2)$) du capteur de gaz à la fin de l'analyse de gaz respiratoire et le signal ($x(t_1)$) du capteur de gaz au début de l'analyse de gaz respiratoire ne dépasse pas une valeur de seuil de quotient pouvant être spécifiée.

4. Procédé selon l'une des revendications 1 à 3, **caractérisé en ce qu'**un critère d'interruption consiste **en ce qu'**une valeur d'un gradient (dx/dt) d'un profil de signal du capteur de gaz ne dépasse pas une valeur de seuil de gradient pouvant être spécifiée.

5. Procédé selon l'une des revendications 1 à 4, **caractérisé en ce qu'**un critère d'interruption consiste **en ce qu'**une valeur d'une dérivée multiple d'un profil de signal du capteur de gaz ne dépasse pas une valeur de seuil de dérivée pouvant être spécifiée.

6. Procédé selon l'une des revendications 1 à 5, **caractérisé en ce qu'**un contrôle (21) du respect du critère d'interruption ou du groupe de critères d'interruption est effectué à des intervalles de temps qui sont choisis (22) respectivement en fonction d'une valeur déterminée en dernier lieu d'au moins un critère d'interruption.

7. Procédé selon l'une des revendications 1 à 6, **caractérisé en ce que** le chauffage (14) est supprimé lorsque le critère d'interruption ou le groupe de critères d'interruption est déjà satisfait à la fin de l'analyse de gaz respiratoire (12).

8. Appareil d'analyse de gaz respiratoire, qui est mis au point pour réaliser un procédé selon l'une des revendications 1 à 7.

## Fig. 1

## Fig. 2

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102011003291 A1
- DE 4107221 A1

- EP 0488102 A2